# EUROPÄISCHE PATENTANMELDUNG

(11) **EP 3 239 121 A1**
(43) Veröffentlichungstag der Anmeldung: **01.11.2017**
(21) Anmeldenummer: 16167796.8
(22) Anmeldetag: 29.04.2016
(51) Int. Cl.: C07C 2/82, C07C 11/02

(54) **VERFAHREN UND ANLAGE ZUR ERZEUGUNG VON OLEFINEN**

(71) Anmelder: Linde Aktiengesellschaft, 80331 München (DE)
(72) Erfinder: Mateos, Daniel, 81373 München (DE); Haidegger, Ernst, 85521 Riemerling (DE); Penner, Florian, 82538 Geretsried (DE)
(74) Vertreter: m patent group

(57) **Zusammenfassung**

Es wird ein Verfahren (100, 200) zur Erzeugung von Olefinen vorgeschlagen, bei dem ein Methan enthaltender erster Reaktionseinsatz zumindest einer oxidativen Kopplung von Methan (2) unterworfen wird, wodurch ein erstes Produktgemisch erhalten wird, das Methan, höhere Kohlenwasserstoffe sowie Wasserstoff, Kohlenmonoxid und Kohlendioxid enthält, wobei unter Verwendung zumindest eines Teils des ersten Produktgemischs eine erste Fraktion, die überwiegend oder ausschließlich Kohlendioxid enthält, und eine zweite Fraktion, die überwiegend oder ausschließlich Methan, Wasserstoff und Kohlenmonoxid enthält, gebildet werden. Es ist vorgesehen, dass unter Verwendung zumindest eines Teils der ersten Fraktion und zumindest eines Teils der zweiten Fraktion ein zweiter Reaktionseinsatz gebildet wird, der einer Oxygenatsynthese (15) unterworfen wird, wodurch ein zweites Produktgemisch erhalten wird, das wenigstens ein Oxygenat enthält, und dass unter Verwendung zumindest eines Teils des zweiten Produktgemischs ein dritter Reaktionseinsatz gebildet wird, der einer Oxygenat-zu-Olefin-Umsetzung (17) unterworfen wird. Eine entsprechende Anlage ist ebenfalls Gegenstand der vorliegenden Erfindung.

## Beschreibung

Die Erfindung betrifft ein Verfahren und eine Anlage zur Erzeugung von Olefinen gemäß den Oberbegriffen der unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet.

Die Reaktionsbedingungen bei der oxidativen Kopplung von Methan umfassen klassischerweise eine Temperatur von 500 bis 900 °C und einen Druck von 1 bis 10 bar sowie hohe Raumgeschwindigkeiten. Jüngere Entwicklungen gehen insbesondere auch in Richtung der Verwendung tieferer Temperaturen. Die Reaktion kann homogen-und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden; der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen beträchtliche Mengen nicht umgesetzten Methans sowie Wasser, Kohlenmonoxid und Kohlendioxid. Ein derartiges Gasgemisch wird nachfolgend auch als "Produktgemisch" der oxidativen Kopplung von Methan bezeichnet, obwohl es nicht nur die gewünschten Produkte, sondern auch nicht umgesetzte Edukte und Nebenprodukte enthält.

Zur Erhöhung der Ausbeute bei der oxidativen Kopplung von Methan wird typischerweise eine Methanisierung von Kohlenmonoxid und teilweise Kohlendioxid eingesetzt, um die Gesamtkohlenstoffausbeute zu erhöhen. Das bei der Methanisierung gebildete Methan kann zusammen mit dem nicht umgesetzten Methan in dem Produktgemisch rezykliert werden. Allerdings sind bei der Methanisierung sehr hohe Mengen an Wasserstoff erforderlich. Zur Methanisierung von Kohlendioxid wird beispielsweise Wasserstoff im Verhältnis von vier zu eins benötigt. Derartig hohe Wasserstoffmengen stehen in dem Produktgemisch der oxidativen Kopplung von Methan nicht zur Verfügung, so dass ein Teil des in Form von Methan eingesetzten Kohlenstoffs in Form von Kohlendioxid als Emission verloren geht.

Es besteht daher der Bedarf nach Verbesserungen in Verfahren, die auf der oxidativen Kopplung von Methan beruhen bzw. entsprechende Verfahrensschritte umfassen, insbesondere im Hinblick auf deren Effizienz und die Gesamtkohlenstoffausbeute.

### Offenbarung der Erfindung

Diese Aufgabe wird durch ein Verfahren und eine Anlage zur Erzeugung von Olefinen mit den Merkmalen der unabhängigen Patentansprüche gelöst. Bevorzugte Ausgestaltungen der vorliegenden Erfindung sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01 % auf molarer, Gewichts- oder Volumenbasis stehen kann. Der Begriff "überwiegend" kann hier insbesondere der soeben getroffenen Definition von "reich" entsprechen.

Ein flüssiges oder gasförmiges Gemisch ist von einem anderen flüssigen oder gasförmigen Gemisch (auch als Ausgangsgemisch bezeichnet) "abgeleitet" oder aus diesem Gemisch oder unter Verwendung dieses Gemischs "gebildet", wenn es zumindest einige in dem Ausgangsgemisch enthaltene oder aus diesem erhaltene Komponenten aufweist. Ein in diesem Sinne gebildetes Gemisch kann aus dem Ausgangsgemisch durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen gebildet werden. Ein "Bilden", beispielsweise eines Reaktionseinsatzes für einen weiteren Prozess, kann jedoch auch einfach das Führen eines entsprechenden Gemischs in einer geeigneten Leitung und ein Zuführen zu dem Prozess darstellen. Wie erwähnt, handelt es sich bei "Produktgemischen" im Sprachgebrauch der vorliegenden Anmeldung um ein mittels eines oder mehrerer Reaktionsschritte erhaltenes Stoffgemisch, das nicht ausschließlich aus den (gewünschten) Produkten bestehen muss, sondern auch Nebenprodukte und nicht umgesetzte Verbindungen aus dem jeweiligen Reaktionseinsatz umfassen kann.

Bei der oxidativen Kopplung von Methan können Reaktoren eingesetzt werden, in denen einer katalytischen Zone eine nichtkatalytische Zone nachgeschaltet ist. Das in der katalytischen Zone gebildete Produktgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Ist nachfolgend davon die Rede, dass ein erster Reaktionseinsatz "zumindest" einer oxidativen Kopplung von Methan unterworfen wird, wodurch ein erstes Produktgemisch gebildet wird, soll hierunter verstanden werden, dass neben der eigentlichen oxidativen Kopplung von Methan auch insbesondere die nichtkatalytischen Schritte, die beim oben erläuterten postkatalytischen Dampfspalten ("Post Bed Cracking") erfolgen, zum Einsatz kommen können. Wird der erste Reaktionseinsatz "zumindest" der oxidativen Kopplung von Methan unterworfen, geht ein Teil des Methans also die zuvor erläuterten Reaktionen ein, insbesondere die Bildung von Methylradikalen, deren Kopplung zu Ethan und die anschließende Oxydehydrierung. Zusätzlich können jedoch Verfahrensschritte umfasst sein, insbesondere das postkatalytische Dampfspalten. Diesem postkatalytischen Dampfspalten oder auch weiteren Verfahren oder Verfahrensschritten können weitere Einsätze zugeführt werden, die zumindest teilweise zu Produkten umgesetzt werden, welche sich in dem ersten Produktgemisch wiederfinden. Mit anderen Worten muss also bei der Bildung des ersten Produktgemischs, die "zumindest" die oxidative Kopplung von Methan umfasst, nicht ausschließlich die oxidative Kopplung von Methan beteiligt sein.

Während kurzkettige Olefine wie Ethylen und Propylen klassischerweise durch das erläuterte Dampfspalten gebildet werden, besteht ein alternativer Weg zu deren Herstellung in den sogenannten Oxygenat-zu-Olefin-Verfahren (engl. Oxygenates to Olefins, OTO), nachfolgend auch als "Oxygenat-zu-Olefin-Umsetzung" bezeichnet. In Oxygenat-zu-Olefin-Verfahren werden Oxygenate wie Methanol oder Dimethylether in eine Reaktionszone eines Reaktors eingebracht, in der ein zur Umsetzung der Oxygenate geeigneter Katalysator bereitgestellt ist. Die Oxygenate werden beispielsweise zu Ethylen und/oder Propylen umgesetzt. Die verwendeten Katalysatoren und Reaktionsbedingungen in Oxygenat-zu-Olefin-Verfahren sind dem Fachmann grundsätzlich bekannt. Je nach dem überwiegend eingesetzten Oxygenat werden entsprechende Verfahren auch als MTO-, MTP- (beim Einsatz von Methanol) oder DTO-Verfahren (beim Einsatz von Dimethylether) bezeichnet.

Oxygenat-zu-Olefin-Verfahren können mit unterschiedlichen Katalysatoren durchgeführt werden. Beispielsweise können Zeolithe wie ZSM-5 oder SAPO-34 oder funktionell vergleichbare Materialien zum Einsatz kommen. Wenn ZSM-5 oder ein vergleichbares Material zum Einsatz kommt, bilden sich vergleichsweise große Mengen an längerkettigen und eher geringe Mengen an kürzerkettigen Kohlenwasserstoffen. Bei der Verwendung von SAPO-34 oder vergleichbaren Materialien werden dagegen eher kürzerkettige Kohlenwasserstoffe gebildet.

Unter Oxygenaten werden gemäß einer gängigen Definition, die auch im vorliegenden Fall Anwendung findet, Verbindungen verstanden, die wenigstens eine kovalent an ein Sauerstoffatom gebundene Alkylgruppe aufweisen. Die wenigstens eine Alkylgruppe kann bis zu fünf, bis zu vier oder bis zu drei Kohlenstoffatome aufweisen. Insbesondere weisen die Oxygenate, die im Rahmen der vorliegenden Erfindung von Interesse sind, Alkylgruppen mit einem oder zwei Kohlenstoffatomen auf, insbesondere handelt es sich um Methylgruppen. Insbesondere handelt es sich um einwertige Alkohole und Dialkylether wie Methanol, Ethanol, tert-Butanol (TBA) und Dimethylether (DME) oder um entsprechende Mischungen. Beispiele für weitere Oxygenate sind Methyl-tert-butylether (MTBE), tert-Amylmethylether (TAME), tert-Amylethylether (TAEE), Ethyl-tert-butylether (ETBE) und Diisopropylether (DIPE). Die Erfindung eignet sich grundsätzlich auch zur Verwendung mit anderen Oxygenaten, wird aber überwiegend unter Bezugnahme auf Methanol und Dimethylether beschrieben.

Die Synthese entsprechender Oxygenate kann insbesondere ausgehend von Synthesegas erfolgen, wie für Methanol beispielsweise im Artikel "Methanol" in Ullmann's Encyclopedia of Industrial Chemistry, Onlineausgabe, 15. Oktober 2012, DOI 10.1002/14356007.a16_465.pub3, beschrieben.

Dimethylether kann durch eine zweistufige Synthese aus Synthesegas über Methanol als Zwischenprodukt gewonnen werden. Entsprechende Verfahren sind beispielsweise ab Seite 171 im DME Handbook des Japan DME Forum, ISBN 978-4-9903839-0-9, 2007, beschrieben. Die zweistufige Gewinnung von Dimethylether aus Synthesegas zeichnet sich dadurch aus, dass zunächst Methanol aus Synthesegas gewonnen wird, anschließend das nicht umgesetzte Synthesegas von den Kondensaten (Methanol und Wasser) abgetrennt wird, und das Methanol schließlich in einem weiteren Reaktor unter Gewinnung von Dimethylether und Wasser dehydratisiert wird. Verfahren zur Gewinnung anderer Oxygenate können in vergleichbarer Weise durchgeführt werden.

In der Patentliteratur wird bereits 1973 (DE 23 62 944 A1, US 4 098 809 A) die direkte oder einstufige Gewinnung von Dimethylether aus Synthesegas beschrieben. Diese zeichnet sich durch eine gemeinsame Reaktionsstufe aus, in der aus Wasserstoff, Kohlenmonoxid und Kohlendioxid gemeinsam Methanol und Dimethylether gewonnen werden. Hierauf aufbauend wurden in der Literatur weitere Verfahren beschrieben. In einem bekannten Kombinationsverfahren, dem sogenannten Topsøe-Prozess, wie er im DME Handbook ab Seite 185, insbesondere auf Seite 187, beschrieben ist, wird ein dualer Katalysator, mit dem sich sowohl Methanol als auch Dimethylether bilden lassen, eingesetzt. Es kommen mindestens zwei Reaktoren ohne zwischengeschaltete Auftrennung zum Einsatz, wobei ein erster Reaktor isotherm gekühlt und ein zweiter Reaktor adiabat betrieben wird. Es erfolgt eine parallele Produktion von Dimethylether und Methanol, wobei das Methanol in einem weiteren Reaktor nach Auftrennung der Komponenten zu Dimethylether umgesetzt werden kann.

Die vorliegende Erfindung kann grundsätzlich mit beliebigen Verfahren zur Oxygenatsynthese und beliebigen Verfahren zur Oxygenat-zu-Olefin-Umsetzung zum Einsatz kommen und ist nicht auf die erläuterten Beispiele beschränkt.

### Vorteile der Erfindung

Im Rahmen der vorliegenden Erfindung wurde erkannt, dass eine Kombination aus einer oxidativen Kopplung von Methan und einer Oxygenat-zu-Olefin-Umsetzung aufgrund der wechselseitigen Synergieeffekte und insbesondere aufgrund der verbesserten Nutzung der bei der oxydativen Kopplung von Methan anfallenden Produktgemische besondere Vorteile bietet. Im Rahmen der vorliegenden Erfindung werden insbesondere die Kohlenstoffausbeute und die Ausbeute an Olefinen erhöht. Diese Vorteile werden nachfolgend erläutert.

Insbesondere wird im Rahmen der vorliegenden Erfindung ausgenutzt, dass sich bei der oxidativen Kopplung von Methan neben den erwünschten Olefinen wie Ethylen und Propylen ein Produktgemisch bildet, das die Komponenten von Synthesegas umfasst bzw. das teilweise als solches verwendet werden kann. Ein entsprechendes Gemisch aus Kohlenmonoxid und Wasserstoff mit Anteilen an Kohlendioxid und nicht umgesetztem Methan kann hinsichtlich seiner Komponenten einzeln oder gemeinsam aus dem Produktgemisch der oxidativen Kopplung von Methan abgetrennt und zur Bereitstellung eines Reaktionseinsatzes für eine Oxygenatsynthese verwendet werden. Hierdurch kann entweder auf die eingangs erwähnte Methanisierung verzichtet werden oder diese kann auf die vorhandenen Wasserstoffmengen begrenzt werden, d.h. nur ein Teil des Kohlenmonoxids bzw. Kohlendioxids in einem Produktgemisch einer oxidativen Kopplung von Methan wird noch der Methanisierung unterworfen.

Ein weiterer Synergieeffekt, der im Rahmen der vorliegenden Erfindung erzielt werden kann, ist der, dass ein Produktgemisch der Oxygenat-zu-Olefin-Umsetzung zu dessen Aufbereitung den ohnehin bereits für die Aufbereitung des Produktgemischs der oxidativen Kopplung von Methan bereitgestellten Aufbereitungsschritten unterworfen werden kann. Auf diese Weise werden sowohl die Erstellungs- als auch die Betriebskosten (CAPEX und OPEX) jedes der getrennten Verfahren bzw. entsprechender Anlagen überproportional reduziert.

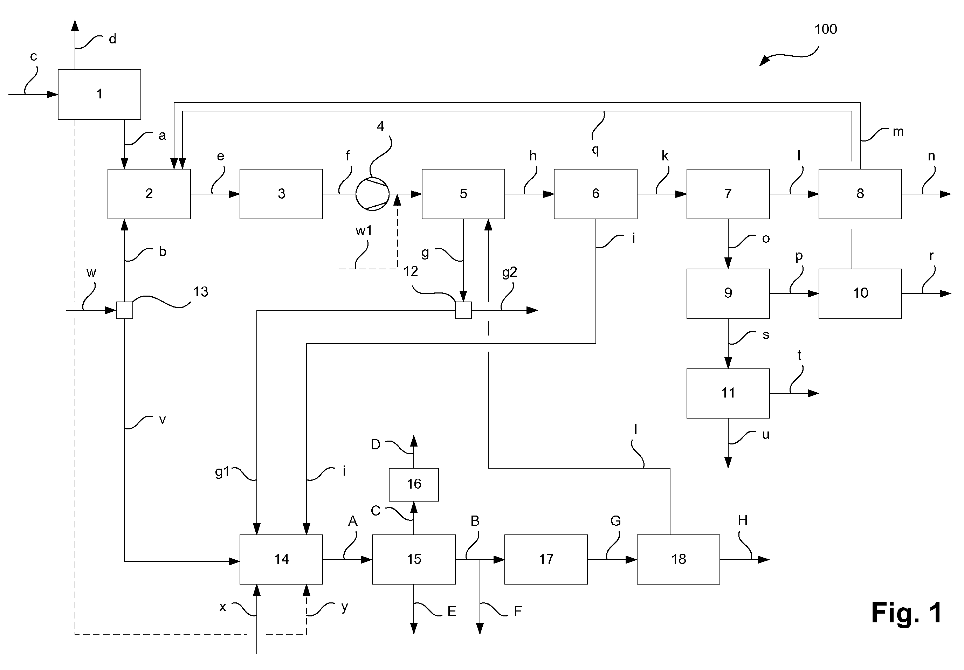

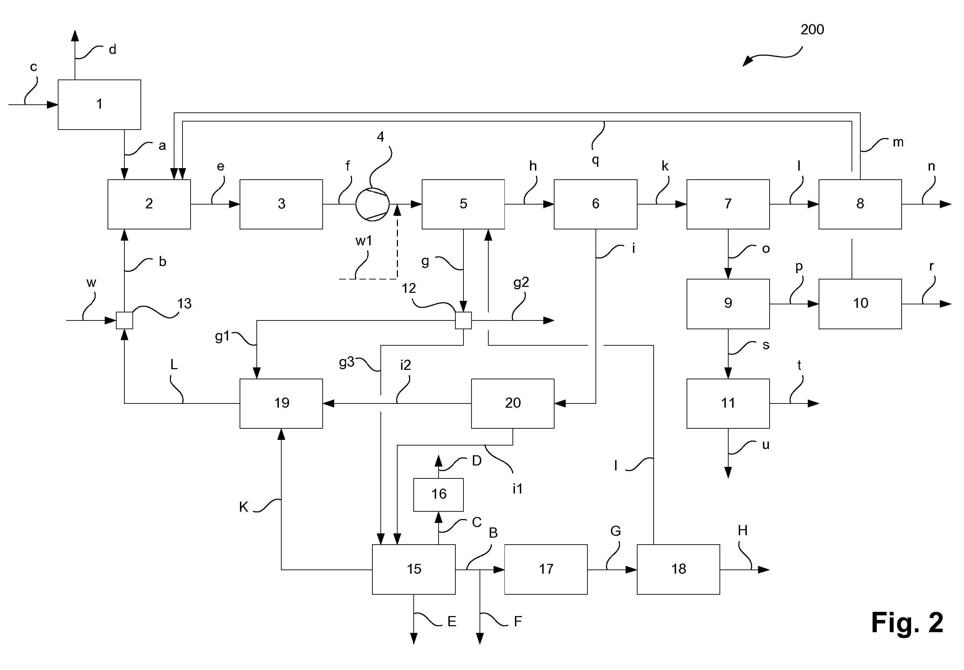

Die vorliegende Erfindung schlägt grundsätzlich zwei auf einer gemeinsamen Idee beruhende Konzepte vor. Beide Konzepte werden auch nachfolgend und insbesondere unter Bezugnahme auf die Figuren 1 und 2 nochmals erläutert.

In einem ersten Konzept wird aus einem Produktgemisch der oxidativen Kopplung von Methan Kohlendioxid und eine Fraktion aus im Wesentlichen Wasserstoff, Methan und Kohlenmonoxid abgetrennt. Sowohl das Kohlendioxid als auch die im Wesentlichen Wasserstoff, Methan und Kohlenmonoxid enthaltende Fraktion können einem Reformierungsverfahren unterworfen werden, in welchem aus dem enthaltenen Methan weiteres Synthesegas gebildet werden kann. Zusätzliches Methan kann der Reformierung ebenfalls zugeführt werden.

Bei der Reformierung kann es sich um ein übliches Reformierungsverfahren handeln, insbesondere um Dampfreformierung oder autotherme Reformierung. Gemäß diesem ersten Konzept wird also das in einem entsprechenden Produktgemisch der oxidativen Kopplung von Methan enthaltene, nicht umgesetzte Methan vollständig zur Erzeugung von Synthesegas genutzt. Das erhaltene Synthesegas kann anschließend einer Oxygenatsynthese, insbesondere einer Methanol- oder Dimethylethersynthese, unterworfen werden.

Gemäß dem zweiten Konzept, das im Rahmen der vorliegenden Erfindung vorgeschlagen wird, wird ebenfalls Kohlendioxid aus dem Produktgemisch der oxidativen Kopplung von Methan abgetrennt, sowie, wie zuvor, die überwiegend oder ausschließlich Wasserstoff, Methan und Kohlenmonoxid enthaltende Fraktion. Letztere wird nun jedoch weiter aufgetrennt. Aus der genannten Fraktion wird eine überwiegend Methan enthaltene Fraktion gebildet, sowie eine überwiegend oder ausschließlich Wasserstoff und Kohlenmonoxid enthaltende Fraktion. Die überwiegend Methan enthaltende Fraktion enthält außerdem Wasserstoff und Kohlenmonoxid, d.h. die bei der Bildung der genannten Fraktionen durchgeführte Trennung ist nicht vollständig. Die überwiegend Methan enthaltende Fraktion kann daher mit einem Teil des abgetrennten Kohlendioxids einer Methanisierung unterworfen werden. Dieser Methanisierung kann auch weiterer Wasserstoff zugeführt werden. Auf diese Weise kann das Kohlendioxid zu Methan umgesetzt werden. Die überwiegend oder ausschließlich Wasserstoff und Kohlenmonoxid enthaltende Fraktion wird hingegen mit dem nicht in der Methanisierung eingesetzten Teil des Kohlendioxids einer Oxygenatsynthese unterworfen. In der Oxygenatsynthese nicht umgesetzter Wasserstoff sowie dort ebenfalls gebildetes Methan und Kohlendioxid können in Mischung als sogenanntes Purge Gas ebenfalls in die Methanisierung geführt werden. Die genannten Komponenten müssen daher vorteilhafterweise nicht getrennt werden.

Insgesamt und konzeptübergreifend schlägt die vorliegende Erfindung ein Verfahren zur Erzeugung von Olefinen vor, bei dem ein Methan enthaltender erster Reaktionseinsatz zumindest ein oxidativen Kopplung von Methan unterworfen wird, wodurch ein erstes Produktgemisch erhalten wird. Wie erläutert, kann bei der Bildung des ersten Produktgemischs neben der eigentlichen oxidativen Kopplung von Methan auch wenigstens ein weiterer Verfahrensschritt umfasst sein, beispielsweise das erläuterte postkatalytische Dampfspalten. Das erste Produktgemisch enthält zumindest Methan, höhere Kohlenwasserstoffe sowie Wasserstoff, Kohlenmonoxid und Kohlendioxid. Unter Verwendung zumindest eines Teils dieses ersten Produktgemischs wird nun eine erste Fraktion gebildet, die überwiegend oder ausschließlich Kohlendioxid enthält, sowie eine zweite Fraktion, die überwiegend oder ausschließlich Methan, Wasserstoff und Kohlenmonoxid enthält.

Erfindungsgemäß wird unter Verwendung zumindest eines Teils der ersten Fraktion, also unter Verwendung zumindest eines Teils des Kohlendioxids aus dem ersten Produktgemisch, und unter Verwendung zumindest eines Teils der zweiten Fraktion, also unter Verwendung zumindest eines Teils des Methans, Wasserstoffs und Kohlenmonoxids, ein Reaktionseinsatz gebildet, der einer Oxygenatsynthese unterworfen wird. Bei der Oxygenatsynthese kann es sich insbesondere um eine Synthese von Methanol und/oder Dimethylether handeln, wie sie eingangs erläutert wurde. Im Rahmen der Oxygenatsynthese wird ein zweites Produktgemisch erhalten, das wenigstens ein Oxygenat enthält. Unter Verwendung zumindest eines Teils des zweiten Produktgemischs wird ein dritter Reaktionseinsatz gebildet, der einer Oxygenat-zu-Olefin-Umsetzung unterworfen wird. Die Synergieeffekte, die durch die erfindungsgemäß vorgeschlagene Integration der oxidativen Kopplung von Methan und der Oxygenat-zu-Olefin-Umsetzung erzielt werden, wurden bereits zuvor erläutert.

Wie ebenfalls bereits erwähnt, werden im Rahmen der vorliegenden Erfindung grundsätzlich zwei Konzepte vorgeschlagen, die sich für die erwähnte Integration besonders eigenen. So kann, gemäß dem bereits erwähnten ersten Konzept, das Bilden des zweiten Reaktionseinsatzes umfassen, Kohlendioxid aus der ersten Fraktion und Methan, Wasserstoff und Kohlenmonoxid aus der zweiten Fraktion einer Reformierung zu unterwerfen, wodurch ein Reformierungsprodukt gebildet wird, das zumindest zum Teil als der zweite Reaktionseinsatz verwendet wird. Dies ist auch insbesondere unter Bezugnahme auf die beigefügte Figur 1 nachfolgend noch erläutert. Die Reformierung kann im Rahmen der vorliegenden Erfindung insbesondere eine Dampfreformierung, eine autotherme Reformierung oder eine partielle Oxidation umfassen. Auch Kombinationen aus Dampfreformierung und autothermer Reformierung bzw. partieller Oxidation können zum Einsatz kommen. Auch die sogenannte Trockenreformierung kann im Rahmen der vorliegenden Erfindung mit Vorteil verwendet werden.

Der Gehalt von Wasserstoff und Kohlenmonoxid in der zweiten Fraktion, die aus dem oben erläuterten Gründen vergleichsweise große Mengen an Methan enthält und nicht, wie ein Einsatz für herkömmliche Reformierungsverfahren, ausschließlich aus Methan besteht, zeigt positive Auswirkungen für- und Betriebskosten bei der Reformierung. In einer entsprechenden Reformierung werden überwiegend Wasserstoff und Kohlenmonoxid erzeugt. Wenn der Reformierung bereits Wasserstoff und Kohlenmonoxid zugeführt werden, reduziert man nicht nur den Methaneinsatz sondern auch die Last für die Reformierung. Ferner kann ein niedrigeres Dampf/Kohlenstoff-Verhältnis eingesetzt werden. Außerdem kann beim Einsatz einer entsprechenden Reformierung das Kohlendioxid vollständig dort genutzt werden, was ebenfalls eine positive Auswirkung auf die gesamte Kohlenstoffeffizienz und die auftretenden Emissionen an Kohlendioxid besitzt. Das Kohlendioxid wird dabei durch eine Wassergasshift zu Kohlenmonoxid umgesetzt.

Ein in der Oxygenatsynthese erhaltenes Wasserstoffprodukt kann, beispielsweise durch Druckwechseladsorption, zurückgewonnen und beispielsweise zur Hydrierung an geeigneter Stelle eingesetzt werden. Stromab der Oxygenat-zu-Olefin-Umsetzung werden Wasser und Oxygenate aus einem dort erhaltenen Produktgemisch entfernt. Das verbleibende Restgemisch, das insbesondere noch Kohlenwasserstoffe enthält, kann in eine thermische Trenneinrichtung geführt werden, die bereits zur Auftrennung der bei der oxidativen Kopplung von Methan gebildeten Kohlenwasserstoffe vorhanden ist. In einer entsprechenden thermischen Trenneinrichtung können beispielsweise Ethylen und Propylen als Produkte gewonnen werden. Auch die Behandlung von Fraktionen aus Kohlenwasserstoffen mit vier sowie fünf und mehr Kohlenstoffatomen und deren Nutzung aus entsprechenden Gemischen ist aus der eingangs erwähnten Fachliteratur bekannt. Paraffine wie Ethan und Propan werden beispielsweise in einem postkatalytischen Dampfspaltschritt stromab der oxidativen Kopplung von Methan zurückgeführt. Die Verwendung einer gemeinsamen Trenneinrichtung erhöht, wie erwähnt, die Synergieeffekte bei der Integration der oxidativen Kopplung von Methan und der Oxygenat-zu-Olefin-Umsetzung.

Je nach dem eingesetzten Reformierungsverfahren können der Reformierung insbesondere weiteres Methan und/oder Wasserdampf und/oder Sauerstoff zugeführt werden. Weiteres Methan kann dann verwendet werden, wenn größere Mengen an Oxygenaten und damit aus Oxygenaten gebildeten Olefine gebildet werden sollen. Insbesondere können für die oxidative Kopplung von Methan und die Reformierung eine gemeinsame Methanquelle eingesetzt werden.

Gemäß dem zuvor erläuterten zweiten Konzept kann das Bilden des zweiten Reaktionseinsatzes jedoch auch umfassen, unter Verwendung zumindest eines Teils der zweiten Fraktion eine Teilfraktion der zweiten Fraktion zu bilden, die überwiegend Wasserstoff und Kohlenmonoxid enthält, wobei zumindest ein Teil der ersten Fraktion und zumindest ein Teil der Teilfraktion der zweiten Fraktion zur Bildung des zweiten Reaktionseinsatzes vermischt werden. Wie bereits erläutert, wird also beispielsweise aus der zweiten Fraktion Methan abgetrennt, wodurch die entsprechende Teilfraktion aus überwiegend oder ausschließlich Wasserstoff und Kohlenmonoxid zurückbleibt. Anstelle des Einsatzes einer Reformierung wird also hier eine zusätzliche Trenneinrichtung eingesetzt. Eine entsprechende Trenneinrichtung kann beispielsweise eine Druckwechseladsorption, ein Membranverfahren oder eine Kombination der genannten Verfahren umfassen. Insbesondere werden hierbei entsprechende Verdichter eingesetzt. Wenngleich durch den Verzicht auf eine Reformierung eine signifikante Kosteneinsparung erzielt wird, die, bezogen auf eine eigenständige Oxygenatanlage, bis zu ca. 40 Prozent betragen kann, entstehen daher durch die zusätzlich erforderliche Trennung Kosten.

Insgesamt werden im Rahmen der vorliegenden Erfindung erhöhte Ausbeuten an Ethylen und Propylen erzielt. Außerdem kommt es zu einer besseren Ausnutzung des Nebenprodukts Synthesegas, das im Rahmen der oxidativen Kopplung von Methan anfällt. Der ökonomische Wert von Synthesegasprodukten liegt typischerweise über den von Methan. Durch die erläuterte Integration werden eine höhere Kohlenstoffausbeute und ein geringerer Energieverbrauch bei reduzierter Größe der erforderlichen Einrichtungen zur Oxygenat-zu-Olefin-Umsetzung erzielt, insbesondere im Vergleich zur Erzeugung von Synthesegas aus Methan. Im Rahmen des zuvor erläuterten ersten Konzepts, d.h. der zusätzlichen Verwendung einer Reformierung, wird das zur Synthese der Olefine erforderliche Methan mengenmäßig reduziert. Zugleich kann theoretisch das gesamte Kohlendioxid in dem ersten Produktgemisch aus der oxydativen Kopplung von Methan in der Dampfreformierung genutzt werden. Auf diese Weise kann das zur Synthese der Olefine erforderliche Methan weiter reduziert werden. Im Rahmen des erläuterten zweiten Konzepts kann auf eine Reformierung verzichtet werden, jedoch sind zusätzliche Trenneinrichtungen bereitzustellen, wie zuvor erläutert.

Die Synergieeffekte im Rahmen der vorliegenden Erfindung umfassen ferner, dass Ethan und Propan bzw. andere Paraffine, die als Nebenprodukte bei der Oxygenatsynthese bzw. der Oxygenat-zu-Olefin-Umsetzung erhalten werden, in ein postkatalytisches Dampfspalten, das stromab des Verfahrens zur oxydativen Kopplung von Methan angeordnet ist, geleitet werden können. Es kann also auf zusätzliche Spaltöfen für ein entsprechendes Dampfspaltverfahren verzichtet werden. Wie erwähnt, kann ein entsprechender Trennteil für sowohl das Produktgemisch aus der oxidativen Kopplung von Methan als auch für jenes der Oxygenat-zu-Olefin-Umsetzung genutzt werden.

Insgesamt ergeben sich im Rahmen der vorliegenden Erfindung deutlich geringere Kohlendioxidemissionen, weil das gebildete Kohlendioxid besser stofflich genutzt werden kann. Bei der herkömmlicherweise ausschließlich verwendeten Methanisierung von Kohlenmonoxid und Kohlendioxid (siehe nachfolgende Reaktionsgleichungen i und ii) sind drei und vier Wasserstoffmoleküle pro Molekül Kohlenmonoxid und Kohlendioxid erforderlich. Bei der Herstellung von Dimethylether sind hingegen lediglich zwei oder drei Wasserstoffmoleküle erforderlich (siehe nachfolgende Reaktionsgleichungen iii und iv):

CO + 3H₂ → CH₄ + H₂O (i) 3 CO + 3 H₂ → DME + CO₂ (iii)

CO₂ + 4H₂ → CH₄ + H₂O (ii) CO₂ + H₂ → CO + H₂O (iv)

Die vorliegende Erfindung ermöglicht es ferner, zusätzlich auf einfache, kosteneffektive Weise weitere Synthesegasprodukte wie Wasserstoff, Methanol, Dimethylether und Ammoniak bzw. Harnstoff herzustellen.

Das Verhältnis von Propylen zu Ethylen kann im Rahmen der vorliegenden Erfindung teilweise durch die verwendeten Reaktionsbedingungen bei der Oxygenat-zu-Olefin-Umsetzung beeinflusst werden.

Im Rahmen des mehrfach erläuterten zweiten Konzepts kann ein erster Anteil der ersten Fraktion, also ein Anteil des Kohlendioxids und zumindest ein Teil der Teilfraktion der zweiten Fraktion zur Bildung des zweiten Reaktionseinsatzes vermischt werden. Eine weitere Teilfraktion der zweiten Fraktion kann gebildet werden, die überwiegend Methan und außerdem Wasserstoff enthält. Diese umfasst also um das abgetrennte Methan aus der zweiten Fraktion. Zumindest ein weiterer Teil der ersten Fraktion und zumindest ein Teil der weiteren Teilfraktion der zweiten Fraktion wird im Rahmen dieser Ausführungsform der vorliegenden Erfindung einer Methanisierung unterworfen, wie bereits oben angesprochen.

Vorteilhafterweise wird ein dabei erhaltenes Methanisierungsprodukt zumindest zum Teil dem ersten Reaktionseinsatz zugegeben. Es wird also weiteres Methan erzeugt, das erneut der oxidativen Kopplung von Methan unterworfen werden kann.

Wie bereits erläutert, kann im Rahmen der vorliegenden Erfindung beispielsweise eine Methanolsynthese und eine Umsetzung des Methanols in der Oxygenat-zu-Olefin-Umsetzung erfolgen. Entsprechendes gilt auch für Dimethylether und dessen Umsetzung zu Olefinen. Wie erläutert, kann mittels der Oxygenatsynthese ferner Wasserstoff gewonnen werden, der aus dem zweiten Produktgemisch abgetrennt und in geeigneter Weise verwendet werden kann. Aus dem zweiten Produktgemisch kann ein Teil des oder enthaltenen Oxygenate abgetrennt und nicht der Oxygenat-zu-Olefin-Umsetzung unterworfen werden. Wie erläutert, können auf diese Weise im Rahmen der vorliegenden Erfindung Nebenprodukte auf einfache und kostengünstige Weise erzeugt und aus dem Verfahren ausgeleitet werden.

Wie ebenfalls erwähnt, kann im Rahmen der vorliegenden Erfindung ein gemeinsamer Trennteil zur Auftrennung von Komponenten des ersten Produktgemischs und eines Produktgemisches der Oxygenat-zu Olefin-Umsetzung verwendet werden. Es kann also in der Oxygenat-zu-Olefin-Umsetzung ein weiteres Produktgemisch gebildet werden, das zumindest zum Teil mit dem ersten Produktgemisch oder einem aus dem ersten Produktgemisch gebildeten Gasgemisch vereinigt wird.

Wie ebenfalls erwähnt, kann der erste Reaktionseinsatz der oxidativen Kopplung von Methan und einem (postkatalytischen) Dampfspaltschritt unterworfen werden, bei der oxidativen Kopplung von Methan nachgeschaltet ist. Der postkatalytische Dampfspaltschritt, wie bereits erläutert, insbesondere in einem gemeinsamen Reaktor mit dem zuvor durchgeführten katalytischen Schritt zur oxidativen Kopplung von Methan eingesetzt werden. Auf diese Weise kann das erste Produktgemisch ebenfalls erhalten werden.

Im Rahmen der vorliegenden Erfindung können die höheren Kohlenwasserstoffe in den ersten Produktgemisch insbesondere Paraffine mit zwei und/oder drei und/oder vier Kohlenstoffatomen sein, wobei diese Paraffine zumindest teilweise aus dem ersten Produktgemisch abgetrennt, und dem Dampfspaltschritt, der der oxidativen Kopplung von Methan nachgeschaltet ist, unterworfen werden können. Entsprechendes gilt auch für derartige höhere Kohlenwasserstoffe aus der Oxygenat-zu-Olefin-Umsetzung. Auf diese Weise kann auf einen Import entsprechender Paraffine zum Einsatz in dem postkatalytischen Dampfspaltschritt verzichtet werden.

Die vorliegende Erfindung erstrecht sich ferner auf eine Anlage zur Erzeugung von Olefinen mit wenigstens einen Reaktor, der dafür eingerichtet ist, einen Methan enthaltenden ersten Reaktionseinsatz zumindest einer oxidativen Kopplung von Methan zu unterwerfen, wodurch ein erstes Produktgemisch erhalten wird, das Methan, höhere Kohlenwasserstoffe sowie Wasserstoff, Kohlenmonoxid und Kohlendioxid enthält, wobei wenigstens eine Trenneinrichtung vorgesehen ist, die dafür eingerichtet ist, unter Verwendung zumindest eines Teils des ersten Produktgemischs eine erste Fraktion, die überwiegend oder ausschließlich Kohlendioxid enthält, und eine zweite Fraktion, die überwiegend oder ausschließlich Methan, Wasserstoff und Kohlenmonoxid enthält, zu bilden, gekennzeichnet durch Mittel, die dafür eingerichtet sind, unter Verwendung zumindest eines Teils der ersten Fraktion und zumindest eines Teils der zweiten Fraktion einen zweiten Reaktionseinsatz zu bilden, wenigstens einen Reaktor, der dafür eingerichtet ist, den zweiten Reaktionseinsatz einer Oxygenatsynthese zu unterwerfen, wodurch ein zweites Produktgemisch erhalten wird, das wenigstens ein Oxygenat enthält, Mittel, die dafür eingerichtet sind, unter Verwendung zumindest eines Teils des zweiten Produktgemischs einen dritten Reaktionseinsatz zu bilden, und wenigstens einen Reaktor, der dafür eingerichtet ist, den dritten Reaktionseinsatz der einer Oxygenat-zu-Olefin-Umsetzung zu unterwerfen.

Eine entsprechende Anlage ist vorteilhafterweise zur Durchführung eines Verfahrens eingerichtet, wie es zuvor im Detail erläutert wurde, und weist hierfür entsprechend eingerichtete Mittel auf. Eine derartige Anlage profitiert daher von den jeweiligen Vorteilen der erläuterten Verfahren bzw. Verfahrensvarianten, so dass auf diese ausdrücklich verwiesen werden kann.

Die Erfindung wird nachfolgend unter Bezugnahme auf die beigefügten Zeichnungen näher erläutert, welche bevorzugte Ausführungsformen der Erfindung zeigen.

### Kurze Beschreibung der Zeichnungen

Figur 1 veranschaulicht ein Verfahren gemäß einer Ausführungsform der Erfindung in Form eines schematischen Ablaufplans.
Figur 2 veranschaulicht ein Verfahren gemäß einer Ausführungsform der Erfindung in Form eines schematischen Ablaufplans.

In Figur ist ein Verfahren gemäß einer Ausführungsform der Erfindung in Form eines schematischen Ablaufplans dargestellt und insgesamt mit 100 bezeichnet. Die dargestellten Elemente bezeichnen auch Komponenten einer entsprechenden Anlage, so dass die nachfolgenden Erläuterungen auch für diese gelten.

Im Rahmen des Verfahrens 100 wird, beispielsweise durch Luftzerlegung 1, ein sauerstoffreicher Stoffstrom a bereitgestellt und einer oxidativen Kopplung von Methan 2 zugeführt. Der oxidativen Kopplung von Methan 2 wird ferner ein methanreicher Einsatz in Form eines Stoffstroms b zugeführt. Der methanreiche Einsatz in Form des Stoffstroms b wird im Rahmen dieser Anmeldung auch als erster Reaktionseinsatz bezeichnet. Der sauerstoffreiche Stoffstrom a kann in der Luftzerlegung 1 aus einem Luftstrom c erzeugt werden. In der Luftzerlegung 1 kann ferner ein stickstoffreicher Strom d erzeugt werden.

Die oxidative Kopplung von Methan 2, die die eingangs erläuterten Reaktionsschritte umfasst, wird ein Produktgemisch erzeugt, das im Rahmen dieser Anmeldung als erstes Produktgemisch bezeichnet wird, und in Form eines Stoffstroms e einer nachfolgenden Kühlung bzw. einem Wasserquench 3 zugeführt werden kann. Wie mehrfach erläutert, kann der eigentlichen oxidativen Kopplung von Methan 2 auch ein postkatalytisches Dampfspalten nachgeschaltet sein, dem geeignete Stoffströme, wie auch nachfolgend noch erläutert, zugeführt werden können. Das in der Kühlung bzw. dem Wasserquench 3 aufbereitete erste Produktgemisch des Stroms e wird in dem hier veranschaulichten Beispiel in Form eines Stroms f einer Verdichtung 4 zugeführt.

Das verdichtete Gasgemisch des Stoffstroms f wird einer Kohlendioxidentfernung und Trocknung 5 unterworfen. In der Kohlendioxidentfernung und Trocknung 5 wird eine kohlendioxidreiche Fraktion abgetrennt und in Form eines Stoffstroms g ausgeführt. Das in der Kohlendioxidentfernung und Trocknung von Kohlendioxid befreite und getrocknete Gasgemisch des Stoffstroms f wird in Form eines Stoffstroms h einer Kühlung und C1/C2-Trennung 6 zugeführt. In der Kühlung und C1/C2-Trennung 6 wird eine, hier als zweite Fraktion bezeichnete Fraktion abgetrennt, die überwiegend oder ausschließlich Wasserstoff, Methan sowie Kohlenmonoxid enthält. Die zuvor erläuterte, überwiegend oder ausschließlich Kohlendioxid enthaltende Fraktion, die in der Kohlendioxidentfernung und Trocknung 5 abgetrennt und in Form des Stoffstroms g ausgeführt wird, wird hingegen hier als erste Fraktion bezeichnet.

Die erläuterte zweite Fraktion wird in Form eines Stroms i abgezogen. Nach der Abtrennung der zweiten Fraktion verbleibt eine Fraktion, die im Wesentlichen Kohlenwasserstoffe mit zwei und mehr Kohlenstoffatomen enthält. Diese kann in Form eines Stoffstroms k in eine C2/C3-Trennung 7 überführt werden. In der C2/C3-Trennung 7 wird eine Fraktion erzeugt, die überwiegend oder ausschließlich Kohlenwasserstoffe mit zwei Kohlenstoffatomen enthält, und die in Form eines Stroms I in eine C2-Hydrierung und C2-Trennung 8 überführt werden kann. Da der Stoffstrom I typischerweise Ethan, Ethylen und Acetylen enthält, kann in der C2-Hydrierung und C2-Trennung 8 das enthaltene Acetylen zu Ethylen hydriert werden. Das in dem Stoffstrom I enthaltene Ethan kann in Form eines Stoffstroms m in die oxidative Kopplung von Methan bzw. in einen entsprechenden postkatalytischen Dampfspaltschritt zurückgeführt werden. Das in dem Stoffstrom I enthaltene Ethylen und das aus der Hydrierung des Acetylens gebildete Ethylen können in Form eines Stoffstroms n als Produkt bereitgestellt werden.

In der C2/C3-Trennung 7 wird ferner eine Fraktion erzeugt, die überwiegend oder ausschließlich Kohlenwasserstoffe mit drei oder mehr Kohlenstoffatomen enthält.

Diese kann in Form eines Stoffstroms o in eine C3/C4-Trennung 9 überführt werden. In der C3/C4-Trennung 9 wird eine Fraktion, die überwiegend oder ausschließlich Kohlenwasserstoffe mit drei Kohlenstoffatomen enthält gebildet. Diese kann in Form eines Stoffstroms p in eine C3-Hydrierung und C3-Trennung 10 überführt werden. In der C3-Hydrierung und C3-Trennung 10 kann, ähnlich zu der C2-Hydrierung und C2-Trennung erläutert, ein überwiegend oder ausschließlich Propan enthaltender Strom q und ein überwiegend oder ausschließlich Propylen enthaltener Strom r erzeugt werden. Der überwiegend oder ausschließlich Propan enthaltende Strom q kann zur oxidativen Kopplung von Methan bzw. einen entsprechenden postkatalytischen Dampfspaltschritt zurückgeführt werden, der überwiegend oder ausschließlich Propylen enthaltende Strom r als Produkt aus dem Verfahren ausgeführt werden.

In der C3/C4-Trennung 9 wird ferner eine überwiegend oder ausschließlich Kohlenwasserstoffe mit vier und mehr Kohlenstoffatomen enthaltende Fraktion gebildet, die in Form eines Stroms s in eine C4-Trennung 11 überführt werden kann. In der C4-Trennung 11 kann eine überwiegend oder ausschließlich Kohlenwasserstoffe mit vier Kohlenstoffatomen enthaltende Fraktion erzeugt und in Form des Stroms t ausgeführt werden. Ferner kann in der C4-Trennung eine überwiegend oder ausschließlich Kohlenwasserstoffe mit fünf und mehr Kohlenstoffatomen enthaltende Fraktion erzeugt und in Form des Stroms u ausgeführt werden.

Das in Figur 1 veranschaulichte Verfahren 100 umfasst ferner eine Reformierung 14, die wie mehrfach erläutert, insbesondere als autotherme Reformierung, Dampfreformierung und/oder partielle Oxidation ausgebildet sein kann. Der Reformierung 14 sind eine Oxygenatsynthese 15, eine Oxygenatumsetzung 17 sowie eine Oxygenataufbereitung 18 nachgeschaltet. Der Oxygenatsynthese 15 ist eine Wasserstoffgewinnung 16 zugeordnet. Der besseren Unterscheidbarkeit halber sind in der Reformierung 14 und den nachgeschalteten Verfahrensschritten gebildete Stoffströme mit Großbuchstaben angegeben.

Wie in Figur 1 veranschaulicht, wird der Reformierung 14 ein überwiegend oder ausschließlich Methan enthaltender Stoffstrom v zugeführt. Der überwiegend oder ausschließlich Methan enthaltende Stoffstrom v kann mittels einer geeigneten Einrichtung 13 aus einem überwiegend oder ausschließlich Methan enthaltenden Ausgangsstrom w gebildet, beispielsweise in die Stoffströme b und v aufgeteilt werden.

Handelt es sich bei dem Stoffstrom w beispielsweise um einen Erdgasstrom, kann dieser auch zum Teil, wie gestrichelt in Form eines Stoffstroms w1 veranschaulicht, stromab der Verdichtung 4 in die Kohlendioxidentfernung und Trocknung 5 geführt werden, wodurch dass sich enthaltene Kohlenwasserstoffe mit zwei und mehr Kohlenstoffatomen gewinnen und entsprechend nutzen lassen.

Ferner wird im dargestellten Beispiel der überwiegend oder ausschließlich Kohlendioxid enthaltende Stoffstrom g in einer entsprechenden Einrichtung 12 in einen ersten Teilstrom g1 und in einen zweiten Teilstrom g2 aufgeteilt. Der Teilstrom g1 und der Stoffstrom i, also ein Teil der mehrfach erwähnten ersten Fraktion und die zweite Fraktion, werden der Reformierung 14 zugeführt, es wird also unter Verwendung dieser Fraktionen oder eines Teils dieser Fraktionen ein entsprechender Reaktionseinsatz gebildet, der hier als zweiter Reaktionseinsatz bezeichnet wird. Der Reformierung 14 kann, je nach Ausgestaltung des dort praktizierten Verfahrens, ein Dampfstrom x und/oder ein Sauerstoffstrom y zugeführt werden. Dieser kann, wie veranschaulicht, ebenfalls aus der Luftzerlegung 1 stammen.

In der Reformierung 14 wird ein Gasgemisch gebildet, das in Form eines Stoffstroms A einer Oxygenatsynthese und Oxygenattrennung 15 zugeführt werden kann. In dieser kann neben einer Oxygenate enthaltenden Fraktion, die in Form eines Stoffstroms B ausgeführt werden kann, eine leichte Fraktion gewonnen und in Form eines Stoffstroms c der Wasserstoffgewinnung 16 zugeführt werden. In der Wasserstoffgewinnung 16 kann ein überwiegend oder ausschließlich Wasserstoff enthaltender Strom D bereitgestellt werden. In der Oxygenatsynthese und Oxygenattrennung 15 kann ferner ein Wasserstrom E bereitgestellt werden.

Der Oxygenate enthaltende Strom B kann teilweise oder vollständig in die Oxygenatumsetzung 17 geführt werden. Beispielsweise kann aus dem Stoffstrom B auch ein Oxygenatstrom F abgetrennt werden. Der in die Oxygenatumsetzung 17 überführte Anteil des Stoffstroms B bzw. die darin enthaltenen Oxygenate, werden in der Oxygenatumsetzung 17 zumindest teilweise zu Olefinen umgesetzt. Ein dabei gebildetes Gasgemisch kann in Form eines Stroms g einer Aufbereitung 18 zugeführt werden, wo die enthaltenen Olefine in Form eines Stroms H neben weiteren Komponenten wie Wasser, abgetrennt werden können. Ein verbleibender Rest, der insbesondere Paraffine umfassen kann, kann beispielsweise der Kohlendioxidentfernung und Trocknung 5 zugeführt werden. Auf diese Weise kann, wie mehrfach erläutert, einer oder mehrere der Trennschritte zur Aufbereitung des Produktgemischs der oxidativen Kopplung vom Methan 2 auch für ein entsprechendes Gasgemisch genutzt werden. Ein entsprechender Strom ist mit I bezeichnet.

In Figur 2 ist ein Verfahren gemäß einer weiteren Ausführungsform der Erfindung in Form eines schematischen Prozessflussdiagramms veranschaulicht und insgesamt mit 200 bezeichnet. Das Verfahren 200 unterscheidet sich von dem Verfahren 100, das in Figur 1 veranschaulicht ist, insbesondere dadurch, dass hier keine Reformierung 14, sondern statt dessen eine Methanisierung 19 und eine Trennung 20 vorgesehen ist. Gleiche oder vergleichbar wie in Figur 1 eingesetzte Ströme mit identischen Bezugszeichen angegeben. Die mehrfach erläuterte zweite Fraktion, also der Strom i, wird in Trennung 20 geführt, in der eine erste Teilfraktion, die überwiegend oder ausschließlich Wasserstoff und Kohlenmonoxid enthält, gebildet wird. Diese wird im dargestellten Beispiel in Form eines Stroms i1 in die Oxygenatsynthese und Oxygenattrennung 15 geführt. Ferner wird in der Trennung 20 eine zweite Teilfraktion gebildet, die überwiegend Methan und außerdem Wasserstoff enthält, und die in Form eines Stroms i2 in die Methanisierung 19 geführt wird.

Der Oxygenatsynthese und Oxygenattrennung 15 wird ferner ein weiterer Teilstrom des Stroms g, also ein weiterer Anteil der ersten Fraktion, zugeführt, und zwar, wie in Figur 2 veranschaulicht, in Form eines Stroms g3. Die in der Oxygenatsynthese und Oxygenattrennung gebildeten Ströme und deren weitere Behandlung ähneln oder gleichen dem Verfahren 100, das in Figur 1 dargestellt ist. Ein sogenanntes Purgegas, das in der Oxygenatsynthese und Oxygenattrennung 15 anfällt, kann in die Methanisierung 19 geführt werden, wie hier in Form des Stroms K veranschaulicht. In Methanisierung 19 wird ein überwiegend oder ausschließlich Methan enthaltender Strom gebildet, wie hier mit Strom I veranschaulicht. Der Strom w wird mit entsprechend geringerer Menge bereitgestellt.

## Patentansprüche

1. Verfahren (100, 200) zur Erzeugung von Olefinen, bei dem ein Methan enthaltender erster Reaktionseinsatz zumindest einer oxidativen Kopplung von Methan (2) unterworfen wird, wodurch ein erstes Produktgemisch erhalten wird, das Methan, höhere Kohlenwasserstoffe sowie Wasserstoff, Kohlenmonoxid und Kohlendioxid enthält, wobei unter Verwendung zumindest eines Teils des ersten Produktgemischs eine erste Fraktion, die überwiegend oder ausschließlich Kohlendioxid enthält, und eine zweite Fraktion, die überwiegend oder ausschließlich Methan, Wasserstoff und Kohlenmonoxid enthält, gebildet werden, **dadurch gekennzeichnet, dass** unter Verwendung zumindest eines Teils der ersten Fraktion und zumindest eines Teils der zweiten Fraktion ein zweiter Reaktionseinsatz gebildet wird, der einer Oxygenatsynthese (15) unterworfen wird, wodurch ein zweites Produktgemisch erhalten wird, das wenigstens ein Oxygenat enthält, und dass unter Verwendung zumindest eines Teils des zweiten Produktgemischs ein dritter Reaktionseinsatz gebildet wird, der einer Oxygenat-zu-Olefin-Umsetzung (17) unterworfen wird.

2. Verfahren (100, 200) nach Anspruch 1, bei dem das Bilden des zweiten Reaktionseinsatzes umfasst, Kohlendioxid aus der ersten Fraktion und Methan, Wasserstoff und Kohlenmonoxid aus der zweiten Fraktion einer Reformierung (14) zu unterwerfen, wodurch ein Reformierungsprodukt gebildet wird, das zumindest zum Teil als der zweite Reaktionseinsatz verwendet wird.

3. Verfahren (100, 200) nach Anspruch 2, bei dem der Reformierung (14) weiteres Methan und/oder Wasserdampf und/oder Sauerstoff zugeführt wird.

4. Verfahren (100, 200) nach Anspruch 1, bei dem das Bilden des zweiten Reaktionseinsatzes umfasst, unter Verwendung zumindest eines Teils der zweiten Fraktion eine Teilfraktion der zweiten Fraktion zu bilden, die überwiegend oder ausschließlich Wasserstoff und Kohlenmonoxid enthält, wobei zumindest ein Teil der ersten Fraktion und zumindest ein Teil der Teilfraktion der zweiten Fraktion zur Bildung des zweiten Reaktionseinsatzes vermischt werden.

5. Verfahren (100, 200) nach Anspruch 4, bei dem ein erster Anteil der ersten Fraktion und zumindest ein Teil der Teilfraktion der zweiten Fraktion zur Bildung des zweiten Reaktionseinsatzes vermischt werden und eine weitere Teilfraktion der zweiten Fraktion gebildet wird, die überwiegend Methan und außerdem Wasserstoff enthält, wobei zumindest ein weiterer Teil der ersten Fraktion und zumindest ein Teil der weiteren Teilfraktion der zweiten Fraktion unter Erhalt eines Methanisierungsprodukts einer Methanisierung (19) unterworfen wird.

6. Verfahren (100, 200) nach Anspruch 5, bei dem das Methanisierungsprodukt zumindest zum Teil dem ersten Reaktionseinsatz zugegeben wird.

7. Verfahren (100, 200) nach einem der vorstehenden Ansprüche, bei dem die Oxygenatsynthese (15) eine Methanolsynthese und die Oxygenat-zu-Olefin-Umsetzung (17) eine Methanol-zu-Olefin-Umsetzung umfasst.

8. Verfahren (100, 200) nach einem der Ansprüche 1 bis 6, bei dem die Oxygenatsynthese (15) eine Dimethylethersynthese und die Oxygenat-zu-Olefin-Umsetzung (17) eine Dimethylether-zu-Olefin-Umsetzung umfasst.

9. Verfahren (100, 200) nach einem der vorstehenden Ansprüche, bei dem mittels der Oxygenatsynthese (15) ferner Wasserstoff gewonnen wird, der aus dem zweiten Produktgemisch abgetrennt wird.

10. Verfahren (100, 200) nach einem der vorstehenden Ansprüche, bei dem aus dem zweiten Produktgemisch ein Teil des oder der enthaltenen Oxygenate abgetrennt und nicht der Oxygenat-zu-Olefin-Umsetzung (17) unterworfen wird.

11. Verfahren (100, 200) nach einem der vorstehenden Ansprüche, bei dem in der Oxygenat-zu-Olefin-Umsetzung (17) ein weiteres Produktgemisch gebildet wird, das zumindest zum Teil mit dem ersten Produktgemisch oder einem aus dem ersten Produktgemisch gebildeten Gasgemisch vereinigt wird.

12. Verfahren (100, 200) nach einem der vorstehenden Ansprüche, bei dem der erste Reaktionseinsatz der oxidativen Kopplung von Methan (2) und einem Dampfspaltschritt, der der oxidativen Kopplung von Methan (2) nachgeschaltet ist, unterworfen wird, wodurch das erstes Produktgemisch erhalten wird.

13. Verfahren nach Anspruch 12, bei dem die höheren Kohlenwasserstoffe in dem ersten Produktgemisch Paraffine mit zwei und/oder drei und/oder vier Kohlenstoffatomen umfassen, wobei diese Paraffine zumindest teilweise aus dem ersten Produktgemisch abgetrennt und dem Dampfspaltschritt, der der oxidativen Kopplung von Methan (2) nachgeschaltet ist, unterworfen wird.

14. Anlage zur Erzeugung von Olefinen, mit wenigstens einem Reaktor, der dafür eingerichtet ist, einen Methan enthaltenden ersten Reaktionseinsatz zumindest einer oxidativen Kopplung von Methan (2) zu unterwerfen, wodurch ein erstes Produktgemisch erhalten wird, das Methan, höhere Kohlenwasserstoffe sowie Wasserstoff, Kohlenmonoxid und Kohlendioxid enthält, wobei wenigstens eine Trenneinrichtung vorgesehen ist, die dafür eingerichtet ist, unter Verwendung zumindest eines Teils des ersten Produktgemischs eine erste Fraktion, die überwiegend oder ausschließlich Kohlendioxid enthält, und eine zweite Fraktion, die überwiegend oder ausschließlich Methan, Wasserstoff und Kohlenmonoxid enthält, zu bilden, **gekennzeichnet durch** Mittel, die dafür eingerichtet sind, unter Verwendung zumindest eines Teils der ersten Fraktion und zumindest eines Teils der zweiten Fraktion einen zweiten Reaktionseinsatz zu bilden, wenigstens einen Reaktor, der dafür eingerichtet ist, den zweiten Reaktionseinsatz einer Oxygenatsynthese (15) zu unterwerfen, wodurch ein zweites Produktgemisch erhalten wird, das wenigstens ein Oxygenat enthält, Mittel, die dafür eingerichtet sind, unter Verwendung zumindest eines Teils des zweiten Produktgemischs einen dritten Reaktionseinsatz zu bilden, und wenigstens einen Reaktor, der dafür eingerichtet ist, den dritten Reaktionseinsatz der einer Oxygenat-zu-Olefin-Umsetzung (17) zu unterwerfen.

15. Anlage nach Anspruch 14, die zur Durchführung eines Verfahrens nach einem der Ansprüche 1 bis 13 eingerichtete Mittel aufweist.
